(19) Europäisches Patentamt
European Patent Office
Office européen des brevets

(11) Veröffentlichungsnummer: **0 071 037**
**B1**

(12) # EUROPÄISCHE PATENTSCHRIFT

(45) Veröffentlichungstag der Patentschrift: **03.10.84**

(51) Int. Cl.³: **C 07 C 91/10**, C 07 C 89/00 // C07C85/11, C07C79/18

(21) Anmeldenummer: **82105912.8**

(22) Anmeldetag: **02.07.82**

(54) **Verfahren zur Herstellung von 2-Aminopropandiol-1,3 (Serinol).**

(30) Priorität: **30.07.81 DE 3130082**

(43) Veröffentlichungstag der Anmeldung: ***09.02.83 Patentblatt 83/6***

(45) Bekanntmachung des Hinweises auf die Patenterteilung: **03.10.84 Patentblatt 84/40**

(84) Benannte Vertragsstaaten: **AT BE CH DE FR GB IT LI LU NL SE**

(56) Entgegenhaltungen:
**DE - B - 2 742 981**
**US - A - 2 174 242**

**BERICHTE DER DEUTSCHEN CHEMISCHEN GESELLSCHAFT, 52. Jahrgang, Band 1, 1919, Berlin E. SCHMIDT et al. "Über einige Derivate des Trimethylenglykols", Seiten 389-399**

(73) Patentinhaber: **DYNAMIT NOBEL AKTIENGESELLSCHAFT, Postfach 1209, D-5210 Troisdorf, Bez. Köln (DE)**
Patentinhaber: **BUSS AG, BASEL, Hohenrainstrasse, CH-4133 Pratteln 1 (CH)**

(72) Erfinder: **Thewalt, Klaus, Dr., Am Tiemen 12, D-5810 Witten (DE)**
Erfinder: **Bison, Günter, Kleiststrasse 6, D-5210 Troisdorf-Sieglar (DE)**
Erfinder: **Egger, Heinz, Lachmattstrasse 19, CH-4132 Muttenz (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

## Beschreibung

Die Erfindung betrifft ein Verfahren zur industriellen Herstellung von 2-Aminopropandiol-1,3 (Serinol).

Serinol ist ein Zwischenprodukt zur Herstellung von Röntgenkontrastmitteln, insbesondere von Iopamidol (N,N'-Bis-[2-hydroxy-1-(hydroxymethyl)äthyl]-2,4-6-trijod-5-lactamidoisophthalamid).

Nach dem Stand der Technik ist die Herstellung von Serinol aus u.a. 2-Oximino-1,3-propandiol, 2-Nitro-1,3-propandiol, Serin, Serinmethylester oder Oximinomalonsäurediäthylester bekannt.

Diese Herstellungsverfahren haben den Nachteil unbefriedigender Ausbeuten und teils schwerer zugängiger Ausgangsstoffe. Es entstehen zersetzliche und gefährliche Nebenprodukte, die nur schwer und aufwendig entfernbar sind. Nach der DE-PS Nr. 2742981 kann Serinol durch Hydrierung des Natriumsalzes des 1,3-Dihydroxy-2-nitropropans, ggf. in Gegenwart einer gepufferten Säure, hergestellt werden. Die Nacharbeitung der dortigen Beispiele bei sowohl 10 als auch 27°C führten zu deutlich geringeren als den angegebenen Ausbeuten. Wiederholungen unter gleichen Bedingungen ergaben Serinol, teils niedrige und sehr stark schwankende Ausbeuten, obwohl die berechnete Menge Wasserstoff stets aufgenommen wurde. Gelegentlich entstehen z.B. 30 oder einmal 50% Serinol, jedoch verhindern wechselnde Mengen labiler Nebenprodukte die Aufarbeitung.

Umsetzungen von 2 kg Na-Nitropropandiol-1,3 in Methanol in Gegenwart von Ammonchlorid mit 0,11 kg 5%igen Pd/C bei 50 bar ergaben einmal trotz langer Hydrierzeiten kein Produkt, das nächste Mal Stoffe, die sich bei Aufarbeitung zersetzten, dann harzartige Kondensationsprodukte. Soweit geringe Mengen Serinol entstanden, waren Nebenprodukte nicht zu entfernen. Temperaturerhöhung auf 30 bis 50°C ergab zwar kürzere Hydrierzeiten, jedoch nahm auch die Bildung harzartiger und labiler Nebenprodukte noch zu.

Im Rührautoklaven ist charakteristisch, dass bei Wiederholung mit gleichen Bedingungen völlig verschiedene Produkte auftreten, folglich die Reproduzierbarkeit deutlich fehlt. Über etwa 25°C erwiesen sich Reaktionen im Rührautoklaven unter allen Bedingungen als unbrauchbar.

Es bestand daher die Aufgabe, Serinol nach einem einwandfrei reproduzierbaren Verfahren mit stets gleichem Erfolg im technischen Massstab in grösseren Mengen und sicheren, wenn möglich erhöhten, Ausbeuten herzustellen.

Gegenstand der Erfindung ist ein Verfahren zur Herstellung von 2-Aminopropandiol-1,3 durch katalytische Hydrierung eines Alkalisalzes des 2-Nitropropandiols-1,3 in Gegenwart inerter Lösungsmittel und einer gepufferten Säure bei erhöhter Temperatur und Drucken von 1 bis 98 bar Wasserstoff, welches dadurch gekennzeichnet ist, dass Temperaturen von 50 bis 80°C mittels einer Kühlvorrichtung während der Reaktion eingehalten werden.

Die Hydrierung erfolgt mit Wasserstoff, der weitere inerte Gase enthalten kann. Geeignete Katalysatoren sind die Platinmetalle Pt, Pd oder Rh, Nickel, Kobalt und Mischkontakte dieser Metalle als Metallpulverniederschläge oder aufgebracht auf Träger. Als gepufferte Säuren werden Salze schwacher Basen mit starken oder mittelstarken Mineralsäuren, besonders Ammonchlorid, -sulfat oder -phosphat verstanden.

Sehr bevorzugt wird die gepufferte Säure in äquivalenten Mengen zugesetzt.

Hierdurch wird, teilweise erst während der Reaktion, aus dem Alkalisalz des Nitropropandiols das Alkali abgespalten, so dass Serinol nach der Formel

$$HO-CH_2-\underset{\underset{O=N-ONa}{\|}}{C}-CH_2-OH \xrightarrow{H_2} HO-CH_2-\underset{\underset{NH_2}{|}}{CH}-CH_2-OH$$

in alkalisierter alkoholischerLösung überraschend letztlich aus dem freien Nitropropandiol entsteht, das sich bei allen Versuchen der Isolierung so leicht zersetzt, dass seine Verwendung als Ausgangsstoff nicht möglich ist.

Die Alkalisalze des Nitropropandiols werden bevorzugt als Suspension eingesetzt.

Sehr bevorzugt ist das Anlagerungsprodukt von 2 mol Alkohol, besonders Methanol, d.h. das sogenannte Azisalz.

Die Reaktion wird in Gegenwart von inerten Lösungsmitteln, besonders Alkoholen mit 1 bis 4 C-Atomen, bevorzugt Methanol, ausgeführt, wobei Wasser anwesend sein kann. Sehr bevorzugt wird die Reaktion unter Einhaltung des engen Temperaturbereichs von 55 bis 60°C während der gesamten Reaktion ausgeführt. Diese Temperatur soll im Semibatch-Verfahren vor Beginn der Reaktion eingestellt werden. Beste Ergebnisse ergibt ein fast isothermer Verlauf, was bei der hohen Reaktionswärme und dem angestrebten raschen Umsatz erhebliche Schwierigkeiten bereitet, die durch rasche Abführung der Wärme beherrscht werden müssen.

Aus unseren Untersuchungen zogen wir die Schlussfolgerung, dass die hohe Reaktionswärme, besonders bei plötzlichem Anspringen der Hydrierung, nur mit einer stark wirkenden Kühlvorrichtung und ggf. einer Dosierung der Ausgangsstoffe während der Reaktion kontrolliert werden kann und andererseits überraschend nur Temperaturen von 50 bis 80°C zum Ziel führen, obgleich gerade bei diesen Temperaturen ohne Kühlvorrichtung nach DBP Nr. 2742981 vollständig oder ganz überwiegend nur Zersetzungsprodukte oder Nebenprodukte entstehen. Ungeeignet sind darunter liegende Temperaturen, die nach der DBP allein geeignet sind, wenn auch nur mit geringen Ausbeuten und fehlender Reproduzierbarkeit, Serinol zu bilden.

Dann jedoch sind auch Umsetzungen pro Charge von 100 bis 2000 kg möglich, ungefährlich und ergeben ganz überlegene Ausbeuten eines Produkts hoher Reinheit.

Wir stellten fest, dass im Batch-Verfahren bei 50 bis 80°C und einer sehr wirksamen Kühlvorrichtung innerhalb der ersten 15 min der Reaktion, besser noch innerhalb von 10 min, mindestens 50, bevorzugt 60 bis 70% oder mehr der Wasserstoffaufnahme zu erfolgen hat, so dass höchste Ansprüche an die Kühlvorrichtung und an die Zugabevorrichtung für den Wasserstoff zu stellen sind.

Als beste Zugabevorrichtung für den Wasserstoff erwies sich eine Mischdüse, die am Ende der vom Reaktor 'ausgehenden und in den Reaktor zurückführenden Ringleitung mit darin liegender Kühleinrichtung angeordnet ist und mit Hilfe grosser Scherkräfte eine innige Vermischung der Reaktionspartner bewirkt.

Bevorzugt ist als stark wirkende Kühlvorrichtung eine ausserhalb des Reaktors liegende Kühlvorrichtung.

Sehr bevorzugt ist eine Kühlvorrichtung in einer vom Reaktor ausgehenden und zu diesem zurückführenden Ringleitung. Die Kühlfläche der Kühlvorrichtung, insbesondere eines gross dimensionalen Wärmeaustauschers ist so zu bemessen, dass bei einer Pumpleistung von 5 bis 70 Volumina des Reaktors je Stunde die Temperatur während der Reaktion, auch während plötzlich und stark einsetzender Reaktion, auf 3 bis 5°C, bevorzugt auf 1 bis 2°C, genau eingehalten werden kann.

Bevorzugt sind Drucke von 20 bis 80 bar.

Als Reaktor mit einer Ringleitung und darin liegender Kühleinrichtung ist besonders ein Schlaufenreaktor geeignet. Schlaufenreaktoren der Bauart BUSS-SR (Hersteller Buss AG, Basel) erwies sich als geeignet.

Ein Schlaufenreaktor ist schematisch in Fig. 1 dargestellt.

Die als Wärmeaustauscher 3 ausgebildete Kühlvorrichtung erlaubt auch das Aufheizen des Ansatzes auf die Reaktionstemperatur vor Beginn der Reaktion.

Mittels der Mischdüse 5 erfolgt die Zugabe des Wasserstoffs und zugleich eine feinste Verteilung des Reaktionsgutes in den Gasraum des Reaktors.

Die Reaktion kann, wenn auch nicht mit gleichem Erfolg, auch mit einem gekühlten Rührautoklaven z.B. einen Hubrührautoklaven ausgeführt werden. Dann ist einerseits für eine grosse Kühlfläche in Form eines Mantelkühlers, eines im Reaktor liegenden Kühlers oder ggf. in einer in einer Ringleitung liegenden Kühlvorrichtung und zugleich für eine gute Durchmischung bei der Reaktion zu sorgen.

Es ist als ausgesprochen überraschend zu bezeichnen, dass nach dem erfindungsgemässen Verfahren die Hydrierung bei Temperaturen von vorwiegend 55 bis 60°C abläuft, weil man nach dem Stand der Technik nicht erwarten konnte, dass diese erhöhte Temperatur die Serinolbildung begünstigt, ohne zugleich die für Temperaturen über 30°C typischen Nebenprodukte in dem Masse zu bilden, wie das bei Ausführung nach den Beispielen der DE-PS Nr. 2742981 der Fall ist. Es muss überraschen, dass bei der Hydrierung im alkalischen Milieu auftretende sehr reaktionsfähige Zwischenstufen, wie beispielsweise Nitroso-, Hydroxylamin-, Azoxy- oder Azoverbindungen, trotz Erhöhung der Temperatur des vorliegenden Verfahrens gegenüber den Beispielen der DE-PS Nr. 2742981 nicht oder in unbedeutender Menge auftreten. Die technischen Vorteile unseres Verfahrens liegen auf der Hand und bestehen besonders in der Umsetzbarkeit grosser Mengen, sehr guter Ausbeuten und Reinheiten, gesicherter Reproduzierbarkeit und damit gefahrloser grosstechnischer Ausführbarkeit. Erschwerende und zeitraubende Reinigungsschritte der Aufarbeitung des Hydrieransatzes sind nicht erforderlich. Besonders ist die in der DE-PS Nr. 2742981 empfohlene Extraktion des Hydriergemisches mit Isopropanol zur Entfernung harziger Nebenprodukte vermeidbar, die dort bei hohen Anteilen von Begleitstoffen schwierig und unwirtschaftlich ist.

Die Beispiele 1 bis 8 betreffen die Ausführung des Verfahrens als Batch-Prozess mit Zugabe aller Stoffe des Ansatzes am Beginn und Aufheizung unter Zugabe von Wasserstoff. Beispiel 9 betrifft einen Semibatch-Prozess mit Dosierung der Stoffe während der Reaktion unter isothermen Bedingungen.

Wasserstoff ist stets im Überschuss vorhanden.

*Beispiel 1*

In einem 1400-l-Schlaufenreaktor werden 680 kg Methanol, 175 kg (844 mol) Natriumsalz von 2-Nitropropandiol-1,3 (× 2 $CH_3OH$), 45,2 kg (844 mol) Ammoniumchlorid und 8,0 kg Katalysator (5% Pd/C, 50% wasserfeucht) eingefüllt. Die Hydrierung wird bei 60 bar Wasserstoff und Raumtemperatur begonnen. Durch Heizen wird der Hydrieransatz innerhalb 10 min auf 55 ± 2°C erwärmt und bei dieser Temperatur gehalten. Innerhalb 15 min werden ca. 75% der theoretischen Menge ≅ 33 m³ Wasserstoff aufgenommen. Die Hydrierung ist nach etwa 3 h beendet. Eine Probe des Ansatzes, gaschromatographisch (GC) analysiert, ergab eine quantitative Hydrierung. Nach Kühlen des Ansatzes auf Raumtemperatur wurde der Katalysator mit dem ausgeschiedenen Kochsalz abfiltriert. Danach wurde im Vakuum bei ~ 300 mbar das ammoniakalische Methanol/Wasser-Gemisch durch Destillation entfernt und der viskose Rückstand vom ausgeschiedenen Kochsalz filtriert. Die anschliessende Destillation über einen Fallfilmverdampfer bei $Kp._{0,5}$ 125-30°C ergab eine Serinolausbeute von 74%, mit einer Reinheit (GC) von 98,7 Gew.-%.

*Beispiele 2 bis 7*

Analog Beispiel 2 wurden die nachstehend in der Tabelle aufgeführten Versuche in einem 50-l-bzw. 1400-l-Schlaufenreaktor durchgeführt:

→

*Beispiel 8*

In einem 750-l-VA-Rührkessel wird ein Gemisch von 50,0 kg (819 mol) Nitromethan, 103,0 Methanol, 50,4 kg (1663 mol) Paraformaldehyd und 0,3 kg 33 gew.-%ige Kalilauge 15 min auf 65°C erhitzt und danach auf 15°C gekühlt. Bei 15 bis 20°C werden in 3 h 166,7 kg (925,8 mol)

| Beispiel Nr. | Ist-Werte | | Einsatzmaterialien | | | | Wasserstoff-aufnahme | | Serinol-gehalt nach GC (%) |
|---|---|---|---|---|---|---|---|---|---|
| | Temp. (°C) | $H_2$-Druck (bar) | Azisalz × 2 $CH_3OH$ (kg) | $CH_3OH$ (kg) | Kat. 5% Pd/C (kg) | $NH_4Cl$ (%) v. Äqui. | Ist ($m^3$) | Soll ($m^3$) | |
| 2 | 52 | 30 | 7,00 | 29,2 | 0,34 | 100 | 1,85 | 2,02 | 79,8 |
| 3 | 55 | 30 | 7,00 | 29,0 | 0,35 | 100 | 1,85 | 2,02 | 84,0 |
| 4 | 58 | 39,5 | 7,00 | 29,0 | 0,35 | 100 | 1,85 | 2,02 | 85,6 |
| 5 | 56 | 60 | 180 | 705 | 8,3 | 100 | 47,0 | 46,8 | 84,5 |
| 6 | 58 | 60 | 180 | 700 | 8,3 | 100 | 47,0 | 46,8 | 94,5 |
| 7 | 65 | 60 | 180 | 700 | 8,3 | 100 | 48,0 | 46,8 | 74,6 |

30 gew.-%ige Natriummethylatlösung zudosiert, wobei sich die Lösung durch das Ausscheiden von 2-Nitropropandiol-1,3 trübt.

Die erhaltene Suspension mit einem theoretischen Gehalt von 161 bis 168 kg 2-Nitropropandiol-1,3 × 2 $CH_3OH$ (Azisalz) wird in einem 1400-l-Schlaufenreaktor gefüllt. Zu dieser Suspension werden 317 kg Methanol, 49,5 kg Ammoniumchlorid und 10,0 kg Katalysator 5% Pd/C (50% wasserfeucht) zugefügt. Diese Mischung wird unter Zugabe von Wasserstoff auf 58°C aufgeheizt,wobei innerhalb von 10 min 70% des Wasserstoffs aufgenommen wurden und unter weiterer Zugabe von Wasserstoff bei 58°C und 65 bar gemäss Beispiel 1 hydriert. Nach ca. 4 h ist die Hydrierung beendet. Die Aufarbeitung gemäss Beispiel 1 ergab bei $Kp._1$ 128 bis 135°C eine gelblich gefärbte viskose Flüssigkeit, die nach einigen Stunden erstarrte. Die Serinolausbeute betrug 75% der Theorie, berechnet auf eingesetztes Nitromethan. Reinheit (GC) 98,7%.

*Beispiel 9*

Gemäss Beispiel 8 wird eine Suspension des 2-Nitropropandiol-1,3 hergestellt, mit 49,5 kg Ammoniumchlorid versetzt und innerhalb 3,5 h unter Hydrierbedingungen des Beispiels 8 in einem 1400-l-Schlaufenreaktor in den Hydrierraum zudosiert, in dem 400 kg Methanol und 10 kg Katalysator 5% Pd/C (50% wasserfeucht) von 58°C vorgelegt waren. Nach beendeter Hydrierung ergab die Analyse (GC) eine mehr als 95%ige Ausbeute. Reinheit 98,8%.

## Patentansprüche

1. Verfahren zur Herstellung von 2-Aminopropandiol-1,3 durch katalytische Hydrierung eines Alkalisalzes des 2-Nitropropandiol-1,3 in Gegenwart inerter Lösungsmittel und einer gepufferten Säure bei erhöhten Temperaturen und Drucken von 1 bis 98 bar Wasserstoff, dadurch gekennzeichnet, dass Temperaturen von 50 bis 80°C mittels einer Kühlvorrichtung während der Reaktion eingehalten werden.

2. Verfahren nach Anspruch 1, dadurch gekennzeichnet, dass die Temperaturen bevorzugt im Bereich von 55 bis 60°C liegen.

3. Verfahren nach einem der Ansprüche 1 oder 2, dadurch gekennzeichnet, dass die Hydrierung in einem temperierbaren Reaktor mit davon ausgehender Ringleitung und Kühlvorrichtung ausgeführt wird.

4. Verfahren nach einem der Ansprüche 1 bis 3, dadurch gekennzeichnet, dass der Wasserstoff über eine Mischdüse, die am Eintritt der Ringleitung in den Reaktor angeordnet ist, zugeführt wird.

5. Verfahren nach mindestens einem der vorangehenden Ansprüche, dadurch gekennzeichnet, dass eine Suspension oder Lösung des Ausgangsstoffes während der Reaktion in den Hydrierraum des Reaktors unter Einhaltung der eingestellten Bedingungen der Temperatur und des Drucks zudosiert wird.

6. Verfahren nach mindestens einem der vorangehenden Ansprüche, dadurch gekennzeichnet, dass im Batch-Prozess innerhalb 15 min nach Reaktionsbeginn mindestens 50% des benötigten Wasserstoffs zugeführt wird.

## Claims

1. Process for the preparation of 2-aminopropanediol-1,3 by catalytic hydrogenation of an alkali salt of 2-nitropropanediol-1,3 in the presence of inert solvent and a buffered acid at elevated temperatures and at pressures of 1 to 98 bar water, characterised in that temperatures of 50 to 80°C are maintained by means of a cooling arrangement during the reaction.

2. Process according to Claim 1, characterised in that the temperatures lie preferably in the region of 55 to 60°C.

3. Process according to one of Claims 1 or 2, characterised in that the hydrogenation is carried out in a reactor capable of being set at a temperature, with closed-circuit pipe-line running therefrom and cooling arrangement.

4. Process according to one of Claims 1 to 3, characterised in that the hydrogen is supplied through a mixing nozzle which is arranged at the entry of the closed-circuit pipe-line into the reactor.

5. Process according to at least one of the preceding claims, characterised in that a suspension or solution of the starting material is metered during the reaction into the hydrogenation chamber of the reactor while maintaining the employed conditions of temperature and of pressure.

6. Process according to at least one of the preceding claims, characterised in that, in the batch process, at least 50% of the required hydrogen is supplied within 15 min of the beginning of the reaction.

**Revendications**

1. Procédé pour la préparation d'amino-2 propanediol-1,3 par hydrogénation catalytique d'un sel alcalin du nitro-2-propanediol-1,3 en présence de solvants inertes et d'un acide tamponné à température élevée et sous des pressions de 0,1 à 9,8 MPa, caractérisé en ce que, pendant la réaction, on maintient des températures de 50 à 80° C au moyen d'un dispositif de refroidissement.

2. Procédé selon la revendication 1, caractérisé en ce que les températures sont comprises de préférence entre 55 et 60° C.

3. Procédé selon l'une des revendications 1 ou 2, caractérisé en ce que l'hydrogénation est réalisée dans un réacteur à température réglable équipé d'une conduite annulaire sortante et d'un dispositif de refroidissement.

4. Procédé selon l'une quelconque des revendications 1 à 3, caractérisé en ce que l'hydrogène est introduit par une buse mélangeuse qui est placée à l'entrée de la conduite annulaire dans le réacteur.

5. Procédé selon au moins l'une des revendications précédentes, caractérisé en ce que l'on introduit une suspension ou une solution de la matière première en quantité dosée pendant la réaction dans la zone d'hydrogénation du réacteur, en maintenant les conditions fixées de température et de pression.

6. Procédé selon au moins l'une des revendications précédentes, caractérisé en ce qu'en procédé discontinu (batch), en 15 min après le début de la réaction, on a introduit au moins 50% de l'hydrogène nécessaire.

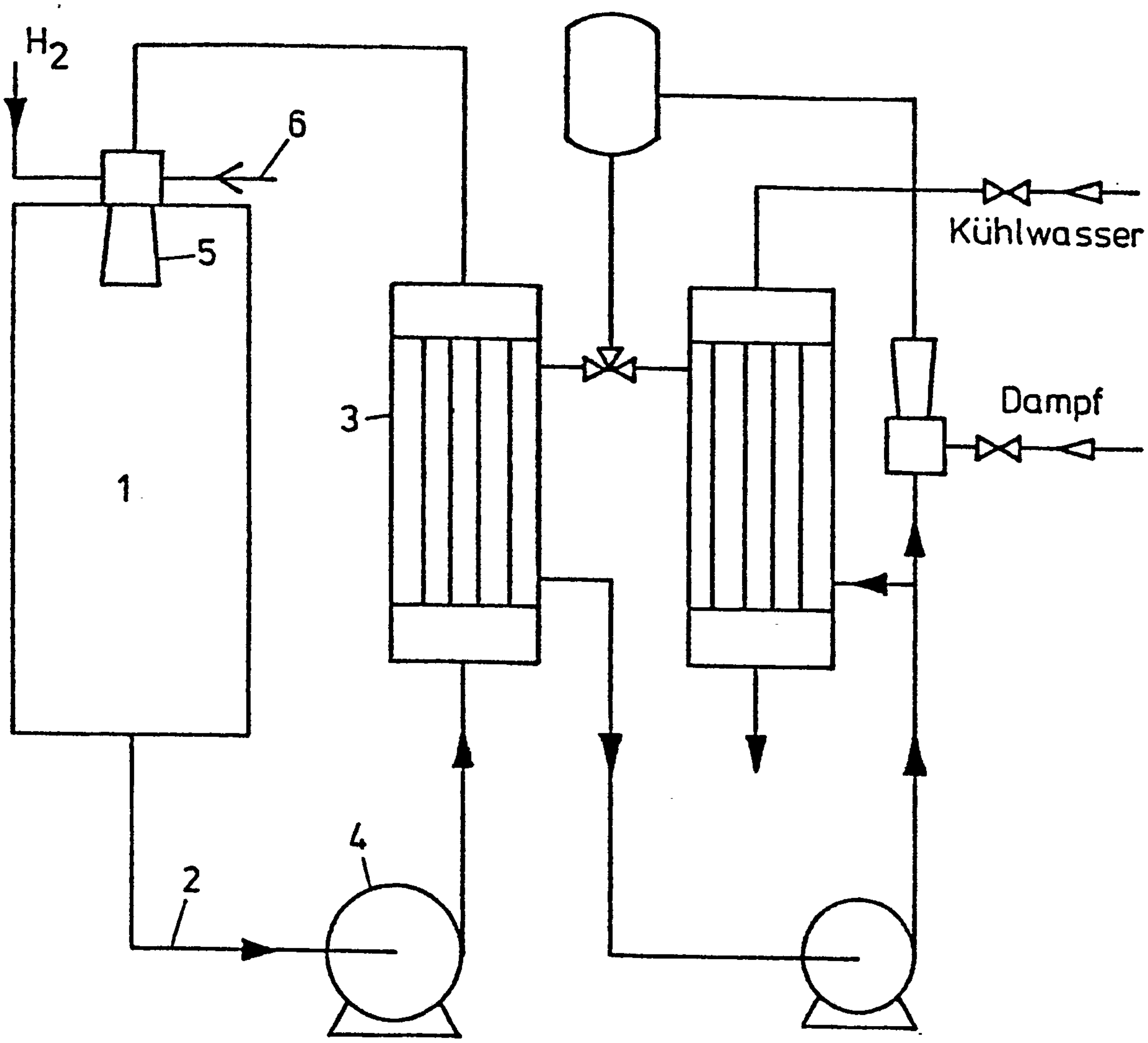


Fig. 1